# EUROPEAN PATENT APPLICATION

(11) **EP 4 752 112 A1**
(43) Date of publication of application: **03.06.2026**
(21) Application number: 25218766.1
(22) Date of filing: 26.11.2025
(51) Int. Cl.: C02F 1/32, E03C 1/126

(54) **SINK DRAINAGE DISINFECTION DEVICE**

(30) Priority: 27.11.2024 LV P2024000071
(71) Applicant: Rigas Tehniska Universitate, 1048 Riga (LV)
(72) Inventor: STRODS, Martins, LV-5041 "Jaunmakoni", Ciemupe, Ogres nov. (LV); DEJUS, Brigita, LV-1002 Zemaisu iela 11-91, Riga (LV); EKKA, Basanti, LV-1083 Tapesu iela 55-25, Riga (LV); RASCEVSKIS, Kristens, LV-1009 Laboratorijas iela 8-4, Riga (LV); SALA, Peteris, LV-5011 Salinas Ledmanes pag., Ogres nov. (LV); JUHNA, Talis, LV- 2164 Alderu iela 30-30 Baltezers, Adazu pag. (LV)
(74) Representative: AAA Law Latvia

(57) **Abstract**

The aim of the invention is to ensure the disinfection of sink drainage, preventing the proliferation of microorganisms, the formation of biofilms, and the entry of microorganisms into the room. The device (1) according to present invention contains the following main components: at least one UV-A light-emitting diode (2), a printed circuit board (4), a radiator (6) for the heat transfer from the UV-A (2) and UV-C light-emitting diodes (38), a fan (8) for cooling the radiator (6), a transparent drainage pipe (20) whose inner surface is coated with a photocatalytic titanium dioxide coating (64), a contour nut (22), at least one UV-C light-emitting diode (38), a programmable controller with a motion sensor (50), as well as a membrane (58) for ensuring one-way movement of liquids and stopping gases from entering the room from the sewage system. The UV-A light-emitting diode (2) and UV-C light-emitting diode (38) provide illumination of the transparent drainage pipe (20) in a perpendicular direction from the outside of the drainage pipe (20) to the inside along its entire perimeter.

## Description

### Field of the invention

The invention relates to the healthcare sector, particularly to halting the growth and spread of microorganisms in discharges from sinks in hospitals and other medical institutions.

### Background of the invention

Since the mid-19th century, when a team of scientists first suggested that hand washing can sharply reduce mortality in pregnant women, hand hygiene has played a key role in preventing and controlling infections [1]. Healthcare-related infections are known to be a major contributor to morbidity and mortality and are often linked to hospital sinks, wastewater outlets, and pipelines [2]. In hospitals, the existing environment, including sinks in hospital wards, can contribute to the spread of pathogens [3]. For example, studies have shown that microorganisms such as vancomycin-resistant enterococci, *Clostridium difficile, Acinetobacter spp.,* methicillin-resistant *Staphylococcus aureus,* and norovirus may appear in hospitals at relatively high concentrations [2]. The presence of these pathogenic microorganisms in hospitals increases the risk of infection. Pathogenic microorganisms are commonly isolated from samples of patients, personnel, and contaminated surfaces [4]. Environmental monitoring also shows that pathogenic microorganisms in hospitals can lead to contamination of medical equipment, which can significantly harm patients' health and staff [5]. Studies conducted on hospital sewage show that contamination with pathogenic microorganisms is primarily formed on sink surfaces and in compounds. Furthermore, the pipelines may produce biofilms that may contain pathogenic microorganisms, which, by facilitating the release of pathogens from the sewer back into the sink or in spray form into the room, may pose a risk to human health. These pathogenic microorganisms are relatively difficult to destroy in sewage pipes. It is, therefore, essential to seek solutions for effective disinfection of sinks and to develop methods to reduce the production of biofilms in pipelines [2].

Several methods are known to reduce microbiological contamination in hospital sinks, including regularly replacing pipes, using various disinfectants, and applying ultraviolet (UV) radiation to antimicrobial and photocatalytic coatings [6-8].

When the semiconductor material is illuminated by UV light, a photocatalytic process occurs, resulting in reactive oxygen species (ROS), a potent oxidizer that can degrade organic molecules, including carbohydrates, nucleic acids, lipids, proteins, and amino acids. Super - peroxide radicals and hydroxyl radicals can also damage different components of bacteria. ROS interactions with lipids cause lipid peroxidation and destroy the integrity of the cell membrane. ROS can also oxidize proteins and nucleic acids in bacterial cells, affecting essential cell functions. The effect of bacterial cell damage caused by ROS is that bacterial cells become dysfunctional and die. Therefore, titanium dioxide (TiO₂) is widely used in semiconductor materials due to its chemical stability, non-toxicity, and relatively low cost [9].

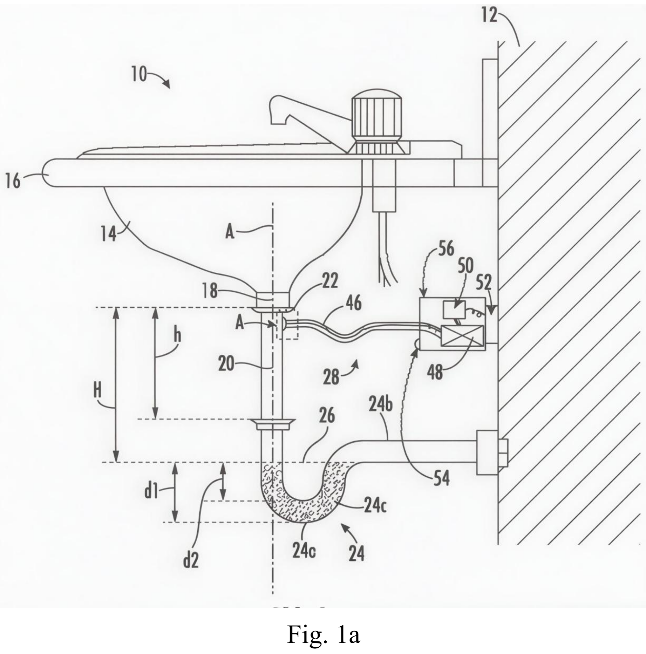

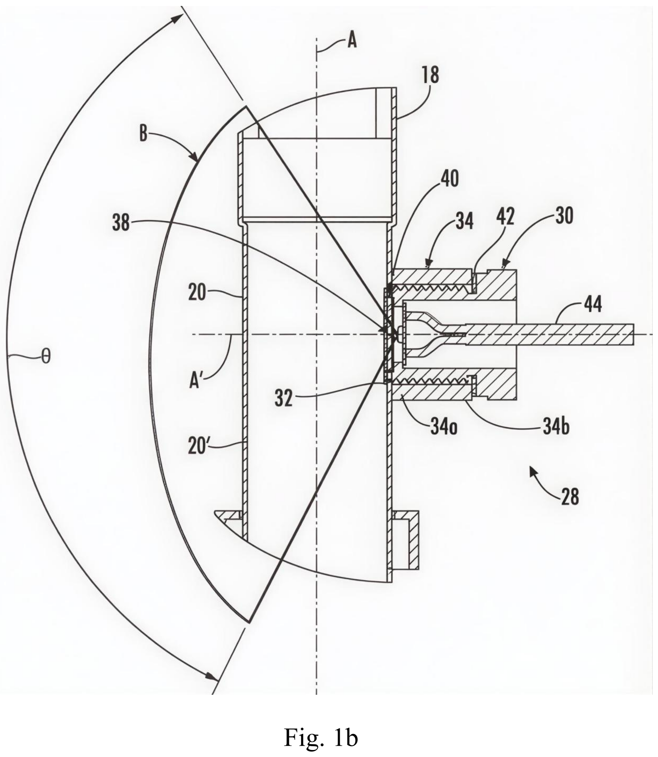

The device known from a prior art (Fig. 1a, 1b) consists of a drainage pipe, a contour nut for connecting the sink support surface and drainage pipe, exhaust disinfection devices, UV-C (light wavelength 200-280 nm) light emitting diodes, UV-C light emitting diodes for the reinforcement of module UV-C LEDs, for the introduction of borehole UV-C radiation into the drainage pipe, threaded sleeves connected to the drainage pipe, transparent lenses placed in the drainage pipe section where the borehole, ring washers seal for provision between threaded sleeve and UV-C LEDs module, electric cable for operation of UV-C LEDs, driver connected to programmable controller with motion sensor providing UV-C LEDs operating mode, and for auxiliary elements: sinks, threaded connection, devices in the wall, sink support surfaces and support panels, exhaust pipes with U-shaped siphon with overflow level, electrical cable protector, 110 V power sockets, and box or housing [10]. This device has been accepted as a prototype of the invention disclosed herein.

For UV-C light diffusion in the above invention prototype device, a transparent lens of a certain design is used, which must be provided as a fixture in the drainage pipe. As shown in Fig. 1b, a transparent lens provides UV radiation only at a certain angle (θ), so there are still places in the drainage tube that are not irradiated and create space for microorganisms to reproduce. In the prototype invention device, the internal surface of the drainage pipe is not coated with photocatalytic titanium dioxide, and a U-shaped siphon has been used. This design prevents gases from entering the room from sewage but does not completely exclude them, thus preventing microorganisms from reproducing and entering the room completely.

Korean patent application KR20160083569A discloses a wash basin drainage pipe sterilization and deodorizing apparatus, as well as a wash basin equipped with the same, capable of effectively suppressing bacterial growth and eliminating unpleasant odors within the drainage pipe. According to one aspect of the invention, said apparatus comprises a drainage pipe, a stopper disposed within the drainage pipe and configured to open and close the drainage channel, an LED positioned within the drainage pipe to emit ultraviolet radiation toward the interior of the pipe, and a photocatalyst layer formed on the stopper and activated by the ultraviolet radiation emitted from the LED.

US patent application US2022040345A1 discloses a system and method for preventing biofouling in the drain pan and drain line of HVAC systems, utilizing one or more UV radiation sources, positioning mechanisms, and one or more UV radiation source control systems.

Korean patent application KR20220096253A discloses a UV LED sterilizer for a sink drain, featuring a circular cover that completely covers the sink drain and a plurality of UV LEDs installed on the inner upper surface of the cover to irradiate UV rays toward the drain hole.

The present invention aims to introduce a novel disinfection device that is intended to prevent the reproduction of microorganisms, the formation of biofilms, and the release of microorganisms into the room, by providing a configuration of a straightforward design than that of prior designs, while remaining compatible with commonly used and readily accessible sewage system connections.

### Purpose and essence of the invention

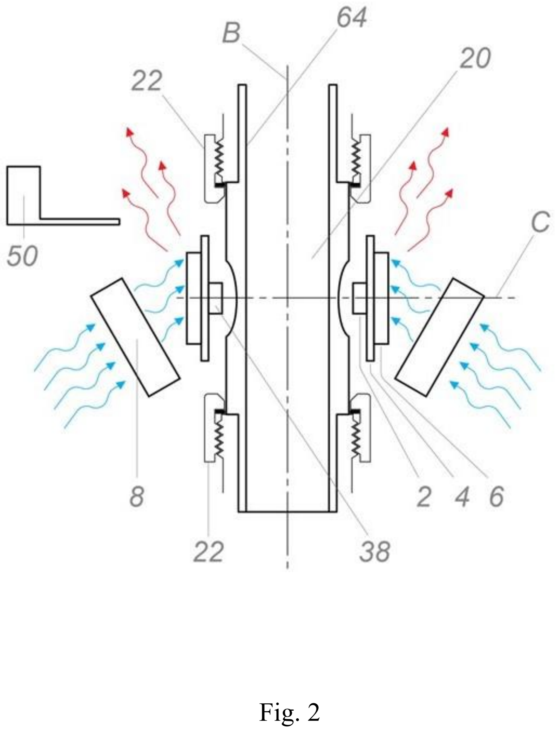

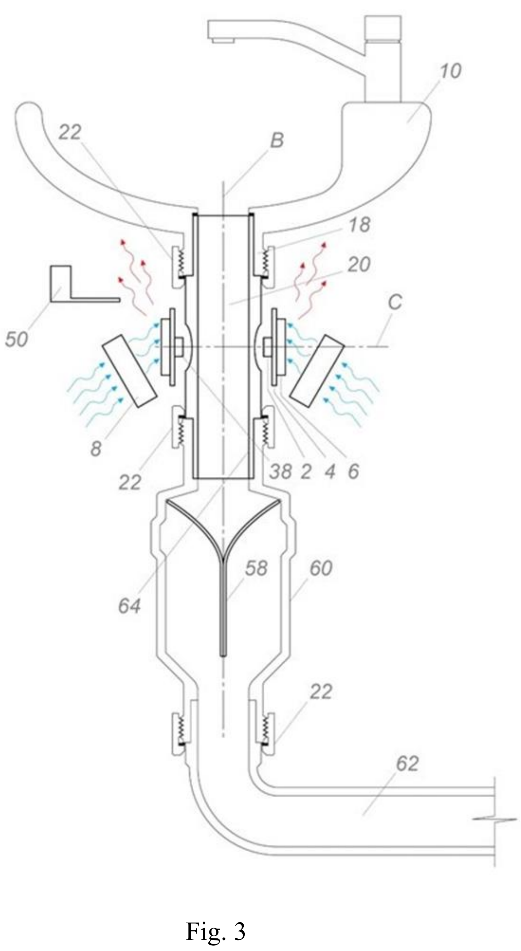

The purpose of the invention has been achieved using a sink discharge disinfection device (hereinafter referred to as the invention device), which includes the following new technical features (Fig. 2 and Fig. 3) as compared to the invention prototype device: sink discharge in addition to UV-C (light wavelength 200-280 nm) light emitting diode (38) equipped with at least one UV-A (light wavelength 320-400 nm) light emitting diode (2); the drainage pipe (20) is transparent and the internal surface of the drainage pipe (20) is coated with photocatalytic titanium dioxide (64); and a membrane (58) that allows one-way movement of fluids and also prevents gas from entering the room from the sewer system. The membrane (58) is secured in a housing (60) connected to a drainage pipe (20) using a contour nut (22). Invention devices for the assembly of water pipes should preferably be used in standard DN40 (DN - nominal diameter (mm) under LVS EN 10255+A1:2009 "non-alloy steel pipes for welding and threading. Technical supply rules) shall measure or other size standardised threaded joints according to the size of the water pipe.

The invention device includes the following key components (Fig. 2 and 3): at least one UV-A light emitting diode (2), printed circuit board (4), radiator (6) for heat transfer from UV-A light emitting diode (2) and UV-C light emitting diode (38), fan (8) for radiator (6) cooling, drainage pipe (20), single contour nut (22) for connecting sink (10) and drainage pipe (20), and one contour nut (22) for connecting membrane (58) housing (60) to drainage tube (20), at least one UV-C LED (38), programmable controller with motion sensor (50) for operating UV-A LEDs (2) and UV-C LEDs (38), drainage tube (20) photocatalytic titanium dioxide coating (64). UV-A LEDs (2) and UV-C LEDs (38) provide a transparent drainage pipe (20) illuminated perpendicularly from the outside of the drainage pipe (20) to the inside throughout its perimeter.

The invention device can be used not only in hospitals but also in other areas where there are problems with the spread of pathogenic micro-organisms in sink discharges, and a standard sink sewer connection and a 220 V electrical connection are available.

The invention device has the following advantages over the invention prototype device:
1. The photocatalytic titanium dioxide coating (64) on the internal surface of the drainage pipe (20) provides a 90 % disinfection level due to UV effects.
2. The invention device does not incorporate a transparent lens (40) (Fig. 1), which complicates the design and manufacture of the invention device, but it has at least one UV-A LED (2) and at least one UV-C LED (38).

The following drawings explain the invention:
Fig. 1a and Fig. 1b illustrate a basic diagram of the device of the prototype invention [10]: drainage tube (20), contour nut (22), exhaust disinfection device (28), UV-C (light wavelength 200-280 nm) LEDs (38), UV-C LEDs (38) module (30), borehole (32), threaded sleeve (34; 34a; 34B), transparent lens (40), ring washer (42), electric cable (44), driver (48), programmable controller with motion sensor (50) and auxiliary elements - sink (10), threaded connection (18), device mounting position in wall (12), sink (10) support surface (14) and support panel (16), exhaust tube with U-shaped siphon (24; 24a; 24B; 24c) with overflow level (26), electrical cable guard (46), 110 V power outlet (52), box or housing (56); H - problem height for microbial distribution, h - length of drainage pipe (20), D1 and D2 - liquid level, B and (θ) - UV-C radiation angles, A - drainage tube (20) symmetry axis, A '- UV-C LEDs (38) module (30) symmetry axis.
Fig. 2. illustrates a basic diagram of the invention device: UV-A (light wavelength 320-400 nm) LEDs (2), printed circuit board (4), radiator (6) for heat transfer from UV-A LEDs (2) and UV-C LEDs (38), fan (8) for radiator (6) cooling, drainage pipe (20), contour nut (22), UV-C LEDs (38), programmable controller with motion sensor (50), photocatalytic titanium dioxide coating (64) on the internal surface of the drainage pipe (20); blue line - ventilation of cold air by fan (8), red line - ventilation of hot air by fan (8), B and C - symmetry axes.
Fig. 3. illustrates a basic diagram of invention device (with connection to sink (10)): UV-A LEDs (2), printed circuit board (4), radiator (6) for heat transfer from UV-A LEDs (2) and UV-C LEDs (38), fan (8) for radiator (6) cooling, sink (10), threaded connection (18), drainage pipe (20), contour nut (22), UV-C light emitting diode (38), programmable controller with motion sensor (50), membrane (58), housing (60), exhaust pipe (62), photocatalytic titanium dioxide coating (64) on the internal surface of drainage pipe (20), axis B and C symmetry.

### Example of the implementation of an invention

Fig. 3 depicts the basic diagram of the invention device (Fig. 2) with connection to the sink (10) - an example of the implementation of this invention is intended for use in hospitals or other medical treatment facilities and includes the following key components: UV-A light emitting diode (2) attached to printed circuit board (4), radiator (6) for heat transfer from UV-A light emitting diode (2) and UV-C light emitting diode (38), fan (8) for radiator (6) cooling, drainage pipe (20) with photocatalytic titanium dioxide coating (64) on the internal surface of drainage pipe (20), sink (10) attached to a wall at a height of 900 millimetres from the floor, threaded connection (18) for connecting drainage pipe (20) to the sink (10) using a contour nut (22), UV-C LEDs (38), programmable controller with motion sensor (50) for operating UV-A LEDs (2) and UV-C LEDs (38), membrane (58) mounted in the housing (60) which is connected to the drainage pipe (20) using a contour nut (22) which ensures one-way movement of liquids, as well as prevents the gas from entering the room from the drainage system, the exhaust pipe (62) connected to the sewer connection of the room. The exhaust pipe (62) shall have a diameter of 50 millimetres. The exhaust pipe (62) is located in the wall, 400 millimeters from the floor, or in the floor.

UV-A light-emitting diode (2) and UV-C light-emitting diode (38) illuminate a drainage tube (20) which is transparent (may be made of organic glass or other transparent material) and the inside of which is coated with photocatalytic titanium dioxide (64), resulting in the release of the drainage tube (20) from unwanted microbiological contamination, if any, as a result of the photocatalytic process. Both UV-A LEDs (2) and UV-C LEDs (38) improve the effectiveness of disinfection by providing 90 % disinfection levels (levels of microbial load and organic pollutant reduction) as the disinfection process combines both direct bactericidal activity and photocatalytic oxidation. The UV-C LEDs (38) are effective at deactivating microorganisms, causing damage to their DNA and RNA, leading to the sequential death of the microorganisms or their inability to reproduce. Consequently, the invention device serves as a bactericidal agent against bacteria, viruses, and other pathogens. On the other hand, the UV-A LEDs (2) play a critical role in activating the photocatalytic titanium dioxide coating (64) on the inside surface of the drainage pipe (20), creating ROS, and then breaking down organic pollutants and cell walls of pathogenic microorganisms.

### References

1. Parkes, L. O., & Hota, S. S. (2018). Sink-Related Outbreaks and Mitigation Strategies in Healthcare Facilities. Current Infectious Disease Reports 20(10). Current Medicine Group LLC 1. Available: https://doi.org/10.1007/s11908-018-0648-3
2. Cole, K., & Talmadge, J. E. (2019). Mitigation of microbial contamination from wastewater and aerosolization by sink design. Journal of Hospital Infection, 103(2), 193-199. Aveilable: https://doi.org/10.1016/j.jhin.2019.05.011
3. Kotay, S., Chai, W., Guilford, W., Barry, K., & Mathers, A. J. (2017). Spread from the sink to the patient: In situ study using green fluorescent protein (GFP)-expressing Escherichia coli to model bacterial dispersion from hand-washing sink-trap reservoirs. Applied and Environmental Microbiology, 83(8). Aveilable: https://doi.org/10.1128/AEM.03327-16
4. Cabral, J., & Rodrigues, A. G. (2019). Blue light disinfection in hospital infection control: Advantages, drawbacks, and pitfalls. Antibiotics 8(2). MDPI AG. Available: https://doi.org/10.3390/antibiotics8020058
5. Lindblad, M., Tano, E., Lindahl, C., & Huss, F. (2020). Ultraviolet-C decontamination of a hospital room: Amount of UV light needed. Burns, 46(4), 842-849. Available: https://doi.org/10.1016/j.burns.2019.10.004
6. Dancer, S. J. (2014). Controlling hospital-acquired infection: Focus on the role of the environment and new technologies for decontamination. Clinical Microbiology Reviews, 27(4), 665-690. Available: https://doi.org/10.1128/CMR.00020-14
7. De Jong, B., Meeder, A. M., Koekkoek, K. W. A. C., Schouten, M. A., Westers, P., & van Zanten, A. R. H. (2018). Pre-post evaluation of effects of a titanium dioxide coating on environmental contamination of an intensive care unit: the TITANIC study. Journal of Hospital Infection, 99(3), 256-262. Available: https://doi.org/10.1016/j.jhin.2017.04.008
8. Memarzadeh, F. (2021). A Review of Recent Evidence for Utilizing Ultraviolet Irradiation Technology to Disinfect Both Indoor Air and Surfaces. Applied Biosafety, 26 (1), 52-56. SAGE Publications Inc. Available: https://doi.org/10.1089/apb.20.0056
9. Pragathiswaran C., Smitha C., Abbubakkar B.M., Govindhan P., Krishnan N.A. (2021). Synthesis and characterization of TiO2/ZnO-Ag nanocomposite for photocatalytic degradation of dyes and anti-microbial activity Materials Today: Proceedings, 45, 3357 - 3364.
10. US11788265B2 STERILUMEN, INC. Interchangeable drain disinfecting device with UV source irradiation optimization. Inventors Munn, Max Tarrytown, NY (US). Available: https://worldwide.espacenet.com/patent/search/family/076545660/publication/US1178826 5B27q=US11788265B2

## Claims

1. Sink discharge disinfection device (1) comprising:
a printed circuit board (4),
a cooling radiator (6),
a drainage pipe (20),
a contour nut (22) for connecting the sink (10) and the drainage pipe (20),
at least one UV-C light-emitting diode (38) and a programmable controller with a motion sensor (50) for operating the UV-C light-emitting diode (38),
**characterized in that** the disinfection device (1) has a fan (8) for the cooling radiator (6), at least one UV-A light emitting diode (2), wherein the drainage tube (20) is of transparent material and has a photocatalytic coating of titanium dioxide (64) applied to the internal surface of the drainage pipe (20).

2. The device according to claim 1, wherein the device (1) has a membrane (58) enclosed in a housing (60), said housing (60) is connected to the drainage pipe (20) using the contour nut (22).

3. The device according to any of the preceding claims, wherein the UV-A light emitting diode (2) illuminates the transparent drainage pipe (20) perpendicular from the outside of the drainage pipe (20) to the inside throughout its perimeter.
